# EUROPEAN PATENT APPLICATION

(11) **EP 4 201 484 A1**
(43) Date of publication of application: **28.06.2023**
(21) Application number: 21850466.0
(22) Date of filing: 19.07.2021
(51) Int. Cl.: A61P 31/12, A61P 43/00, C12N 15/113, C12N 15/57, C12N 9/48, A61K 31/7088, A61K 31/7115

(54) **ANTISENSE NUCLEIC ACID INDUCING EXON SKIPPING OF ANGIOTENSIN CONVERTING ENZYME 2 GENE**

(30) Priority: 28.07.2020 JP 2020127142
(71) Applicant: KNC Laboratories Co., Ltd., Kobe, Hyogo 650-0047 (JP); Kobe Gakuin Educational Foundation, Kobe-shi, Hyogo 650-8586 (JP)
(72) Inventor: MATSUO, Masafumi, Kobe-shi, Hyogo 651-2180 (JP); MAETA, Kazuhiro, Kobe-shi, Hyogo 650-0047 (JP); FUJIHARA, Tsuyoshi, Kobe-shi, Hyogo 650-0047 (JP); OKAMOTO, Itaru, Kobe-shi, Hyogo 650-0047 (JP); NEYA, Masahiro, Kobe-shi, Hyogo 650-0047 (JP)
(74) Representative: Hoffmann Eitle
(86) International application number: PCT/JP2021/026930
(87) International publication number: WO 2022/024833

(57) **Abstract**

An antisense oligonucleotide is provided which induces exon skipping in ACE2 gene. An antisense oligonucleotide of 15-30 bases or a salt or a solvate thereof, wherein the antisense oligonucleotide has a nucleotide sequence complementary to a target site in exon 18 of angiotensin-converting enzyme 2 gene and is capable of inducing exon skipping in angiotensin-converting enzyme 2 gene. A pharmaceutical drug comprising the antisense oligonucleotide or a salt or a solvate thereof; and an agent for inhibiting the expression of angiotensin-converting enzyme 2 protein and/or for enhancing the expression of soluble angiotensin-converting enzyme 2.

## Description

### TECHNICAL FIELD

The present invention relates to an antisense oligonucleotide inducing exon skipping in angiotensin-converting enzyme 2 gene.

### BACKGROUND ART

Angiotensin-converting enzyme 2 (ACE2) is a type I transmembrane protein of approximately 92 kDa consisting of 805 amino acids and is encoded by ACE2 gene of 107 kb located at locus Xp22 on X chromosome. The cDNA of ACE2 gene is approximately 3 kb in size and consists of 19 exons (NM_021804.3). ACE2 has a hydrolysis action of cutting off the C-terminal amino acid of peptides, and converts angiotensin I and angiotensin II to angiotensin 1-9 and angiotensin 1-7 (Ang(1-7)), resepectively, as a carboxypeptidase.

On the other hand, during the epidemic of severe acute respiratory syndrome (SARS) in 2002 to 2003, it was revealed that ACE2 is a receptor for coronavirus SARS-CoV entering into the body, and ACE2 attracted attention as a key molecule for the establishment of viral infection. Further, it has been revealed that SARS-CoV-2 currently spreading as COVID-19 throughout the world also uses ACE2 as its receptor (Non-Patent Document No. 1).

ACE2 is a transmembrane protein consisting of three domains, extracellular, transmembrane and intracellular, with the extracellular domain containing a virus-binding domain. This virus-binding domain binds to SARS-CoV-2 with high affinity to thereby bear the function of a receptor for the virus. Therefore, ACE2 has become one of the targets of SARS-CoV-2 therapy and various methods such as a method of inhibiting the expression of ACE2 and a method in which a soluble ACE2 consisting of an extracellular domain alone is used as a decoy receptor have been studied intensively (Non-Patent Document No. 2).

Splicing is a reaction which produces a mature mRNA composed of exons alone by cutting off introns from pre-mRNA transcribed from a gene. Splicing sites are determined by GT-AG sequences called splicing consensus sequences that are located at both ends of introns. In addition to these sequences, splicing enhancers act as cis elements, leading to accurate splicing reaction. Antisense oligonucleotides (ASOs) to the splicing enhancers inhibit the splicing enhancer function to thereby induce exon skipping. Development of ASOs that induce exon skipping is ongoing positively for treating hereditary diseases or the like (Non-Patent Document No. 3).

### PRIOR ART LITERATURE

### Non-Patent Documents

Non-Patent Document No. 1:
   Hoffmann M, Kleine Weber H, Schroeder S, Kruger N, Herrler T, Erichsen S, et al. Hoffmann M, Kleine Weber H, Schroeder S, Kruger N, Herrler T, Erichsen S, et al. SARS-CoV-2 Cell Entry Depends on ACE2 and TMPRSS2 and Is Blocked by a Clinically Proven Protease Inhibitor. Cell. 2020; 181(2):271-80.e8.
Non-Patent Document No. 2:
   Hodgson J. The pandemic pipeline. Nat Biotechnol. 2020;38(5):523-32.
Non-Patent Document No. 3:
   Masafumi Matuo, Perspective of Antisense Oligonucleotide-Mediated Exon-Skipping Therapies, Journal of Nucleic Acids Therapeutics Society of Japan 5, 4-13 (2020)

### DISCLOSURE OF THE INVENTION

### PROBLEM FOR SOLUTION BY THE INVENTION

The present inventors have conceived application of this exon skipping to ACE2 gene. ACE2 gene consists of 19 exons, with exon 18 encoding a transmembrane domain. When exon 18 is skipped, 195 bases are eliminated from the mRNA, which results in an ACE2 protein shorter by 65 amino acids. This protein is a novel, soluble ACE2 protein lacking the transmembrane domain, and is expected to act as a decoy receptor for the virus. Therefore, the skipping of exon 18 which leads to both a decrease of the receptor for SARS-CoV-2 and an increase of the decoy receptor is greatly expected to be a powerful and breakthrough therapy for preventing virus infection.

After the present inventors started research and development of the subject antisense oligonucleotide, Rehman et al. gave an illustration by exon skipping in ACE2 gene using ASO in in-silico study (Rehman S, and Tabish M. Alternative splicing of ACE2 possibly generates variants that may limit the entry of SARS-CoV-2: a potential therapeutic approach using SSOs. Clin Sci (Lond). 2020;134(10):1143-5).

The object of the present invention is to provide an antisense oligonucleotide (ASO) capable of inducing exon skipping in ACE2 gene for the purposes of inhibiting the expression of ACE2 protein and enhancing the expression of soluble ACE2.

### MEANS TO SOLVE THE PROBLEM

The present inventors have identified antisense oligonucleotides (ASOs) that induce the skipping of exon 18 of ACE2 gene.

A summary of the present invention is described as below.
(1) An antisense oligonucleotide of 15-30 bases or a salt or a solvate thereof, wherein the antisense oligonucleotide has a nucleotide sequence complementary to a target site in exon 18 of angiotensin-converting enzyme 2 gene and is capable of inducing exon skipping in angiotensin-converting enzyme 2 gene.
(2) The antisense oligonucleotide or a salt or a solvate thereof of (1) above, wherein the nucleotide sequence of exon 18 of angiotensin-converting enzyme 2 gene is the nucleotide sequence as shown in SEQ ID NO: 1, and the target site in exon 18 of angiotensin-converting enzyme 2 gene is located within the region of nucleotide Nos. 1-195 of the nucleotide sequence as shown in SEQ ID NO: 1.
(3) The antisense oligonucleotide or a salt or a solvate thereof of (1) or (2) above, wherein the nucleotide sequence of the antisense oligonucleotide comprises a sequence consisting of at least 15 consecutive nucleotides in any one of the sequences as shown in SEQ ID NOS: 2-17 (wherein "t" may be "u", and "u" may be "t").
(4) The antisense oligonucleotide or a salt or a solvate thereof of any one of (1) to (3) above, wherein the antisense oligonucleotide has 18 bases.
(5) The antisense oligonucleotide or a salt or a solvate thereof of (4) above, wherein the nucleotide sequence of the antisense oligonucleotide is any one of the sequences as shown in SEQ ID NOS: 2-17 (wherein "t" may be "u", and "u" may be "t").
(6) The antisense oligonucleotide or a salt or a solvate thereof of any one of (1) to (5) above, wherein at least one nucleotide is modified.
(7) The antisense oligonucleotide or a salt or a solvate thereof of (6) above, wherein the sugar constituting the modified nucleotide is D-ribofuranose and the hydroxy group at 2'-position of D-ribofuranose is modified.
(8) The antisense oligonucleotide or a salt or a solvate thereof of (7) above, wherein D-ribofuranose is 2'-O-alkylated and/or 2'-O,4'-C-alkylenated.
(9) A pharmaceutical drug comprising the antisense oligonucleotide of any one of (1) to (8) above or a pharmaceutically acceptable salt or solvate thereof.
(10) The pharmaceutical drug of (9) above for inhibiting the infectivity of SARS-CoV-2.
(11) The pharmaceutical drug of (10) above, which has an effect of inhibiting cell entry of the virus by decreasing receptor-type angiotensin-converting enzyme 2 and/or an effect of extracellular capturing of the virus by increasing soluble angiotensin-converting enzyme 2 capable of binding to the virus.
(12) The pharmaceutical drug of any one of (8) to (11) above for preventing and/or treating SARS-CoV-2 infection.
(13) An agent for inhibiting the expression of angiotensin-converting enzyme 2 protein and/or enhancing the expression of soluble angiotensin-converting enzyme 2, wherein the agent comprises the antisense oligonucleotide or a salt or a solvate thereof of any one of (1) to (8) above.
(14) A soluble angiotensin-converting enzyme 2 lacking its transmembrane domain, which is produced by exon skipping in angiotensin-converting enzyme 2 gene, said exon skipping being induced by the antisense oligonucleotide or a salt or a solvate thereof of any one of (1) to (8) above.
(15) A polynucleotide comprising a nucleotide sequence encoding the soluble angiotensin-converting enzyme 2 of (14) above and/or a sequence complementary to said sequence.
(16) A method of inhibiting the infectivity of SARS-CoV-2, comprising administering to a subject an effective amount of the antisense oligonucleotide or a pharmaceutically acceptable salt or a solvate thereof of any one of (1) to (8) above.
(17) A method of preventing and/or treating SARS-CoV-2 infection, comprising administering to a subject an effective amount of the antisense oligonucleotide or a pharmaceutically acceptable salt or a solvate thereof of any one of (1) to (8) above.
(18) A method of inhibiting the expression of angiotensin-converting enzyme 2 protein and/or enhancing the expression of soluble angiotensin-converting enzyme 2, comprising administering to a subject an effective amount of the antisense oligonucleotide or a pharmaceutically acceptable salt or a solvate thereof of any one of (1) to (8) above.
(19) The antisense oligonucleotide or a pharmaceutically acceptable salt or a solvate thereof of any one of (1) to (8) above, for use in a method of inhibiting the infectivity of SARS-CoV-2.
(20) The antisense oligonucleotide or a pharmaceutically acceptable salt or a solvate thereof of any one of (1) to (8) above, for use in a method of preventing and/or treating SARS-CoV-2 infection.
(21) The antisense oligonucleotide or a pharmaceutically acceptable salt or a solvate thereof of any one of (1) to (8) above, for use in a method of inhibiting the expression of angiotensin-converting enzyme 2 protein and/or enhancing the expression of soluble angiotensin-converting enzyme 2.
(22) Use of the antisense oligonucleotide or a pharmaceutically acceptable salt or a solvate thereof of any one of (1) to (8) above in the manufacture of a pharmaceutical drug for inhibiting the infectivity of SARS-CoV-2.
(23) Use of the antisense oligonucleotide or a pharmaceutically acceptable salt or a solvate thereof of any one of (1) to (8) above in the manufacture of a pharmaceutical drug for preventing and/or treating SARS-CoV-2 infection.
(24) Use of the antisense oligonucleotide or a pharmaceutically acceptable salt or a solvate thereof of any one of (1) to (8) above in the manufacture of an agent for inhibiting the expression of angiotensin-converting enzyme 2 protein and/or enhancing the expression of soluble angiotensin-converting enzyme 2.

### EFFECT OF THE INVENTION

Skipping of exon 18 of ACE2 gene yields an mRNA molecule from which 195 nucleotides encoded by exon 18 have been removed. As a result, an ACE2 protein shorter by 65 amino acids is produced. It should be noted here that the entire amino acid sequence constituting the transmembrane domain is contained in these 65 amino acids. This means that a soluble ACE2 lacking the transmembrane domain is produced. Consequently, expression of the receptor for SARS-CoV-2 infection is inhibited; a decoy receptor is expressed; and a peptidase is expressed. Thus, it is expected that a substantial effect of preventing SARS-CoV-2 infection can be obtained.

The present specification encompasses the contents disclosed in the specification and/or the drawings of Japanese Patent Application No. 2020-127142 based on which the present patent application claims priority.

### BRIEF DESCRIPTION OF THE DRAWINGS

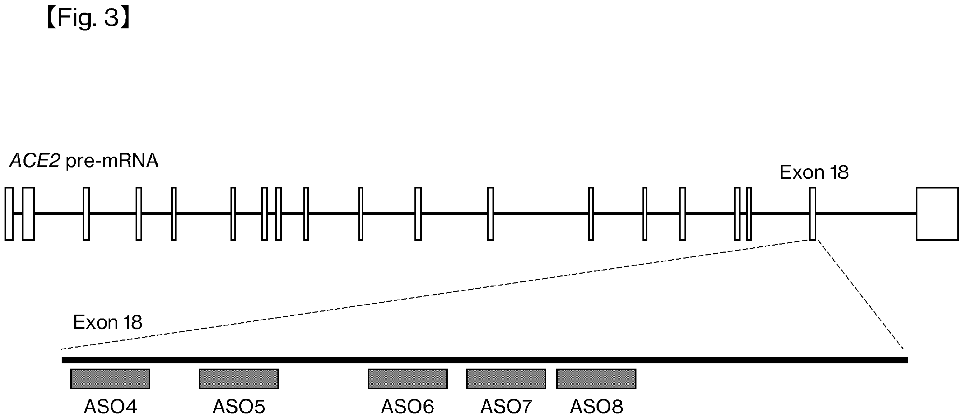

[Fig. 1] ACE2 pre-mRNA and Target Sites of ASOs
   ACE2 pre-mRNA which is transcribed and produced from the ACE2 gene comprises 19 exons. For the purpose of skipping exon 18, the present inventors prepared antisense oligonucleotides (ASO1, ASO2 and ASO3) complementary to sequences within exon 18 of ACE2.
[Fig. 2] Comparison of Efficacies of ASOs
   Each ASO was introduced into human liver carcinoma cells (HepG2), and the resultant ACE2 mRNA was analyzed by RT-PCR. The results are shown. a) Results of RT-PCR on ACE2 mRNA and GAPDH mRNA of human liver carcinoma cells (HepG2) are shown. "w/o" means without ASO treatment. White arrowhead represents ACE2. Black arrowhead represents exon 18-skipped ACE2. b) From the results of RT-PCR, the ratio of exon 18-skipped ACE2 mRNA to total ACE2 mRNA was determined. Three independent experiments were conducted and the results were summarized. *P<0.05
[Fig. 3] ACE2 pre-mRNA and Target Sites of ASOs
   Further, in order to search for ASOs effective for the skipping of exon 18, the present inventors prepared ASO4, 5, 6, 7 and 8 complementary to sequences within exon 18 of ACE2.

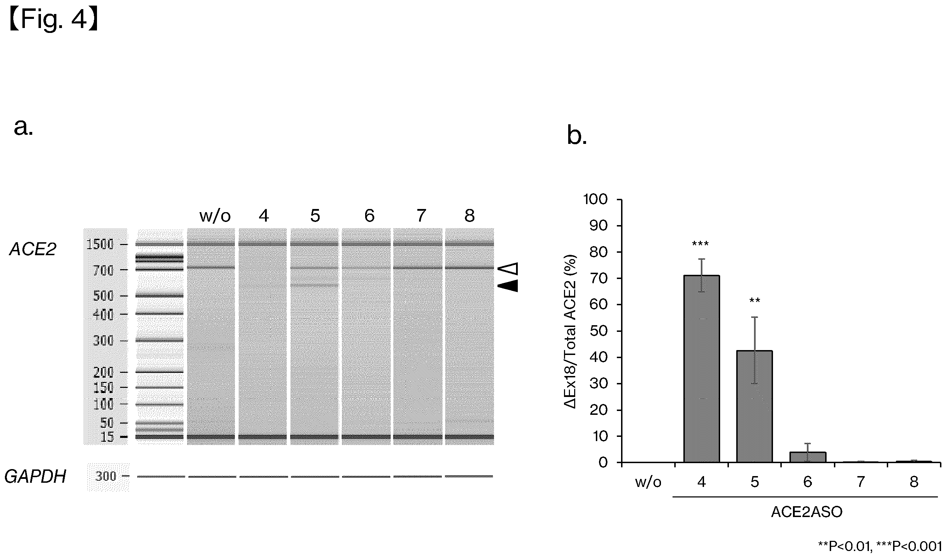

[Fig. 4] Comparison of Efficacies of ASOs
   Each of ASO4, 5, 6, 7 and 8 was introduced into human liver carcinoma cells (HepG2), and the resultant ACE2 mRNA was analyzed by RT-PCR. The results are shown. a) Results of RT-PCR on ACE2 mRNA and GAPDH mRNA of human liver carcinoma cells (HepG2) are shown. "w/o" means without ASO treatment. White arrowhead represents ACE2. Black arrowhead represents exon 18-skipped ACE2. b) From the results of RT-PCR, the ratio of exon 18-skipped ACE2 mRNA to total ACE2 mRNA was determined. Three independent experiments were conducted and the results were summarized. **P<0.01. ***P<0.001.

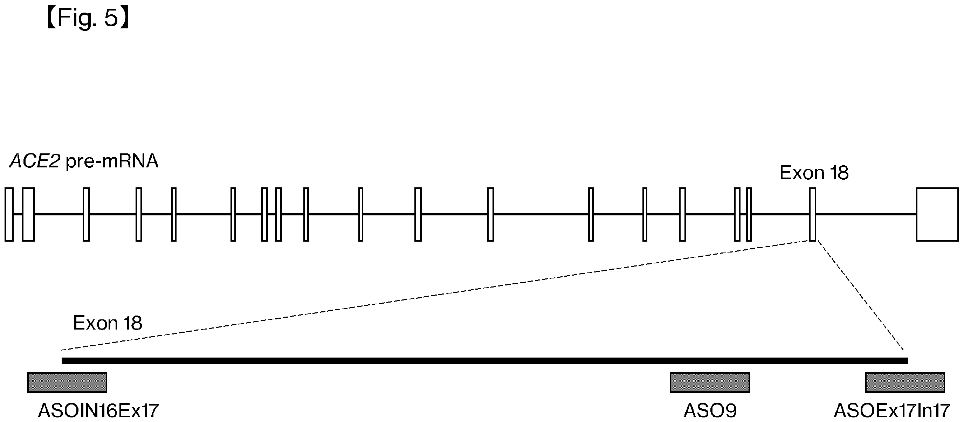

[Fig. 5] ACE2 pre-mRNA and Target Sites of ASOs
   Further, in order to search for ASOs effective for the skipping of exon 18, the present inventors prepared ASO9, ASOIn16Ex17 and ASOEx17In17 complementary to sequences in exon 18 and/or introns flanking exon 18 of ACE2.

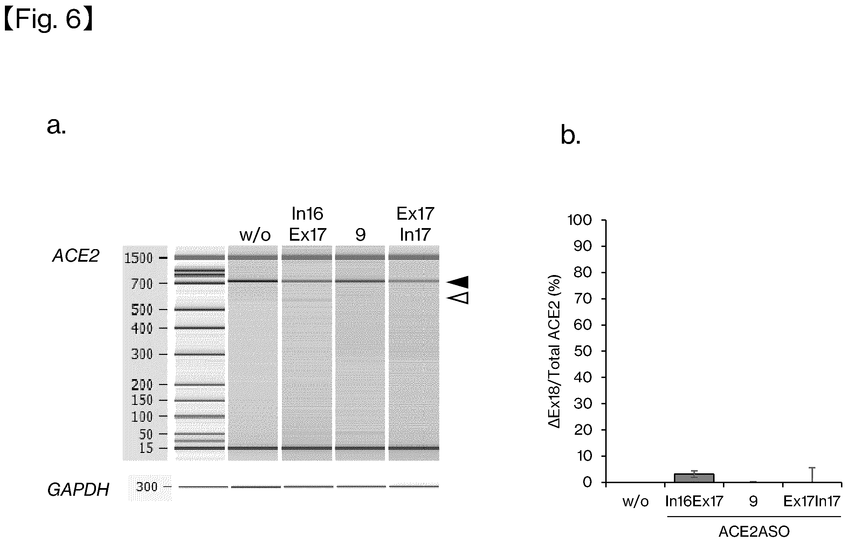

[Fig. 6] Comparison of Efficacies of ASOs
   Each of ASO9, In16Ex17 and Ex17In17 was introduced into human liver carcinoma cells (HepG2), and the resultant ACE2 mRNA was analyzed by RT-PCR. The results are shown. a) Results of RT-PCR on ACE2 mRNA and GAPDH mRNA of human liver carcinoma cells (HepG2) are shown. "w/o" means without ASO treatment. Black arrowhead represents ACE2. White arrowhead represents exon 18-skipped ACE2. b) The ratio of exon 18-skipped ACE2 mRNA to total ACE2 mRNA is shown. Three independent experiments were conducted and the results were summarized.

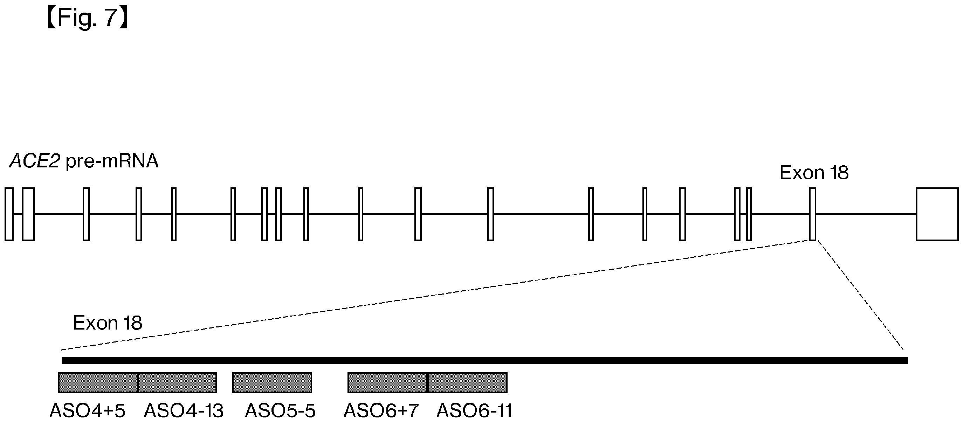

[Fig. 7] ACE2 pre-mRNA and Target Sites of ASOs
   In order to search for ASOs effective for the skipping of exon 18 further, the present inventors have prepared ASO4+5, 4-13, 5-5, 6+7 and 6-11 complementary to sequences in exon 18 or intron 17 of ACE2.

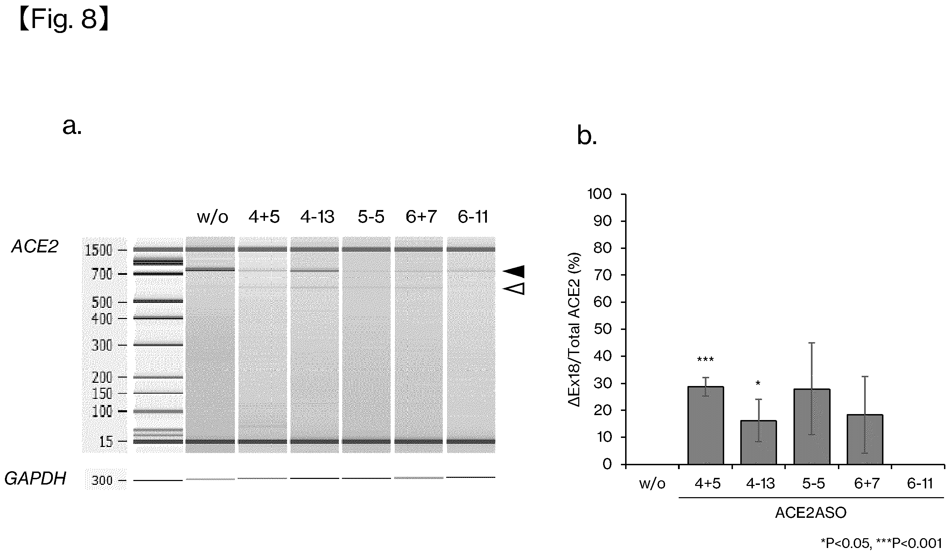

[Fig. 8] Comparison of Efficacies of ASOs
   Each of ASO4+5, 4-13, 5-5, 6+7 and 6-11 was introduced into human liver carcinoma cells (HepG2), and the resultant ACE2 mRNA was analyzed by RT-PCR. The results are shown. a) Results of RT-PCR on ACE2 mRNA and GAPDH mRNA of human liver carcinoma cells (HepG2) are shown. "w/o" means without ASO treatment. Black arrowhead represents ACE2. White arrowhead represents exon 18-skipped ACE2. b) The ratio of exon 18-skipped ACE2 mRNA to total ACE2 mRNA is shown. Three independent experiments were conducted and the results were summarized. *P<0.05, ***P<0.001.

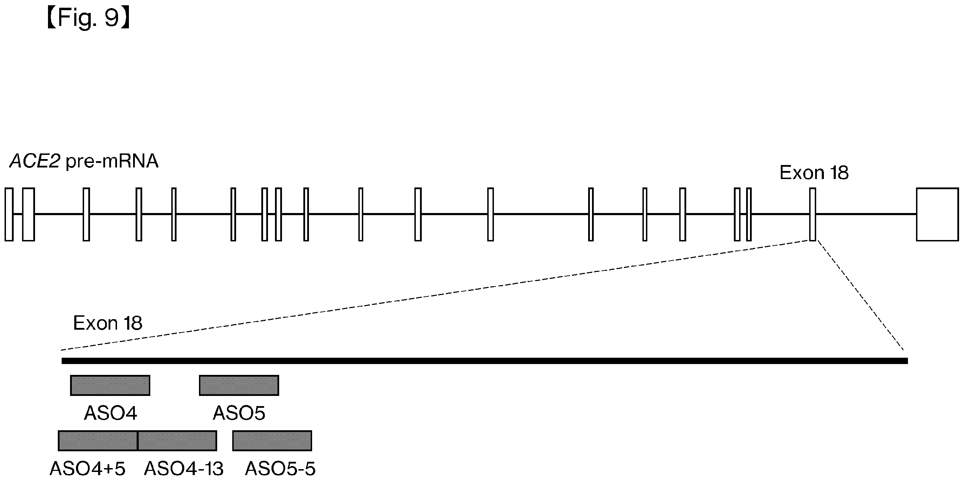

[Fig. 9] ACE2 pre-mRNA and Target Sites of ASOs
   In view of the results of examination conducted 4 times on the efficacies of ASOs as described above, 5 ASOs which exhibited a high skipping efficacy on exon 18 were selected and their efficacies were compared.

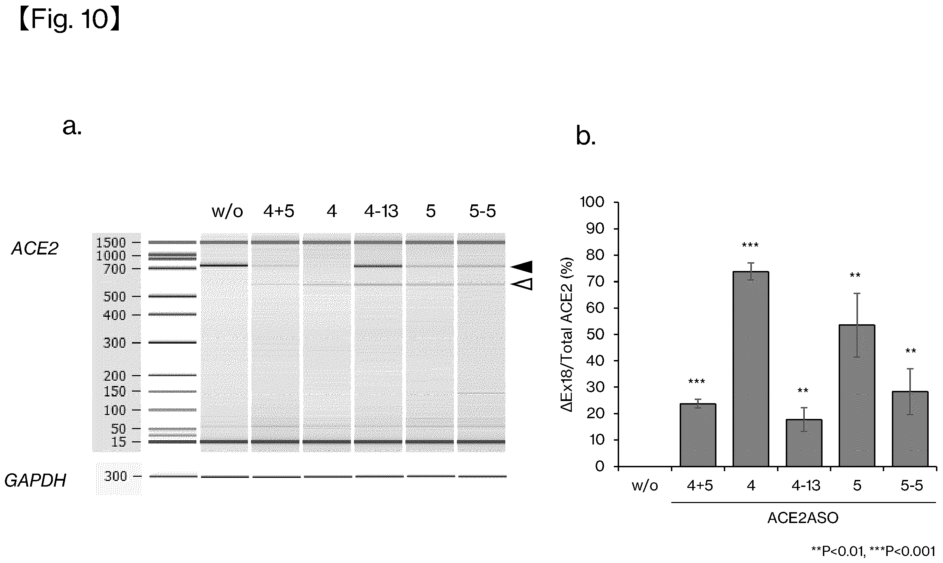

[Fig. 10] Comparison of Efficacies of ASOs
   Each of the 5 ASOs was introduced into human liver carcinoma cells (HepG2), and the resultant ACE2 mRNA was analyzed by RT-PCR. The results are shown. a) Results of RT-PCR on ACE2 mRNA and GAPDH mRNA of human liver carcinoma cells (HepG2) are shown. "w/o" means without ASO treatment. Black arrowhead represents ACE2. White arrowhead represents exon 18-skipped ACE2. b) The ratio of exon 18-skipped ACE2 mRNA to total ACE2 mRNA is shown. Three independent experiments were conducted and the results were summarized. *P<0.05, ***P<0.001.

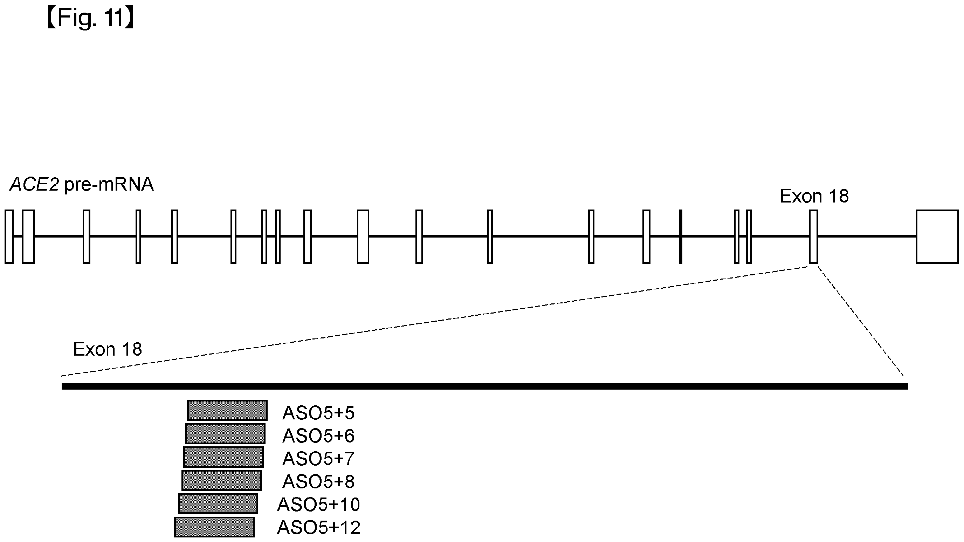

[Fig. 11] ACE2 pre-mRNA and Target Sites of ASOs
   Further, in order to search for ASOs effective for the skipping of exon 18, the present inventors prepared ASO5+5, 5+6, 5+7, 5+8, 5+10 and 5+12 complementary to sequences within exon 18 of ACE2.

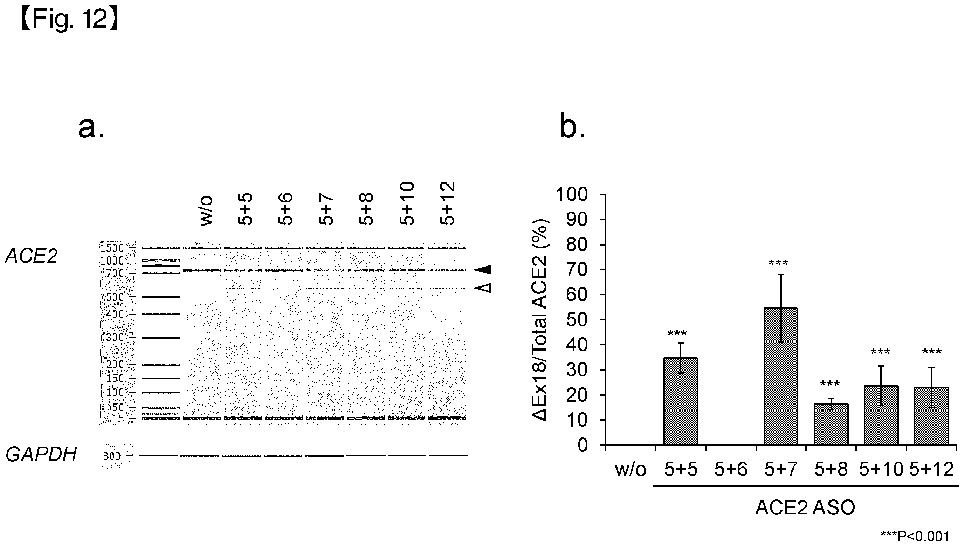

[Fig. 12] Comparison of Efficacies of ASOs
   Each of ASOS+5, 5+6, 5+7, 5+8, 5+10 and 5+12 was introduced into human liver carcinoma cells (HepG2), and the resultant ACE2 mRNA was analyzed by RT-PCR. The results are shown. a) Results of RT-PCR on ACE2 mRNA and GAPDH mRNA of human liver carcinoma cells (HepG2) are shown. "w/o" means without ASO treatment. Black arrowhead represents ACE2. White arrowhead represents exon 18-skipped ACE2. b) The ratio of exon 18-skipped ACE2 mRNA to total ACE2 mRNA is shown. Three independent experiments were conducted and the results were summarized. ***P<0.001.

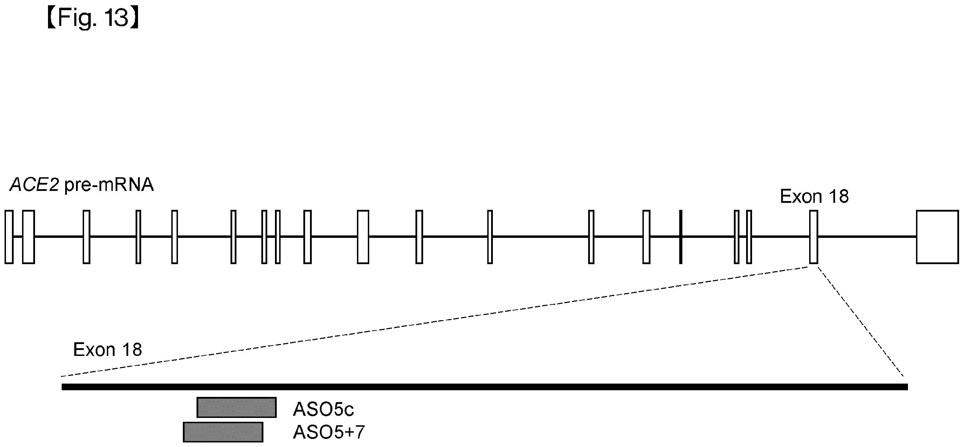

[Fig. 13] ACE2 pre-mRNA and Target Sites of ASOs
   In order to search for ASOs that were the most effective for the skipping of exon 18, the present inventors prepared ASO5+7 and ASO5c complementary to a sequence within exon 18 of ACE2. Although the target sequence of ASOSc is the same as that of ASOS, the positions of ENA^{®} (2'-O,4'-C-Ethylene-bridged Nucleic Acids) and 2'OMe (2'-O-methyl-RNA) in ASO are different from those in ASOS.

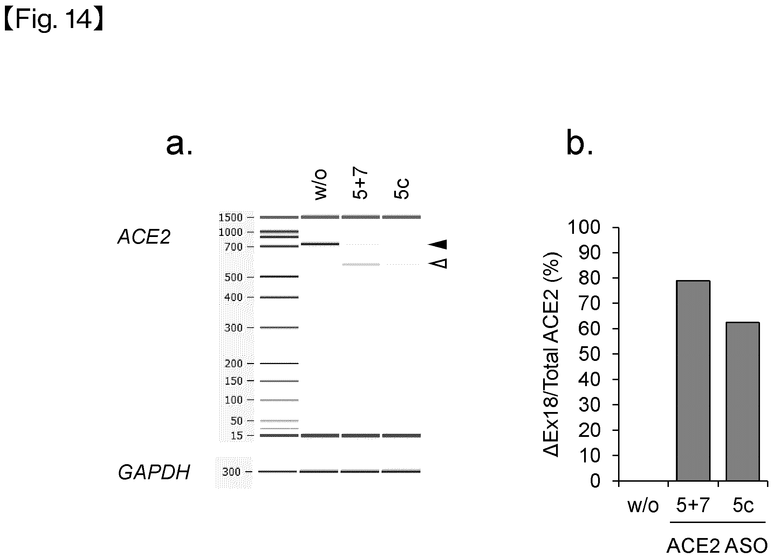

[Fig. 14] Comparison of Efficacies of ASOs
   Each of ASO5+7 and ASOSc was introduced into human liver carcinoma cells (HepG2), and the resultant ACE2 mRNA was analyzed by RT-PCR. The results are shown. a) Results of RT-PCR on ACE2 mRNA and GAPDH mRNA of human liver carcinoma cells (HepG2) are shown. "w/o" means without ASO treatment. Black arrowhead represents ACE2. White arrowhead represents exon 18-skipped ACE2. b) The ratio of exon 18-skipped ACE2 mRNA to total ACE2 mRNA is shown. Two independent experiments were conducted and the results were summarized. ASO5+7 was shown to be the most effective for the skipping of ACE2 exon 18 in HepG2 cells.

### BEST MODES FOR CARRYING OUT THE INVENTION

Hereinbelow, embodiments of the present invention will be described in detail.

The present invention provides an antisense oligonucleotide of 15-30 bases or a salt or a solvate thereof, wherein the antisense oligonucleotide has a nucleotide sequence complementary to a target site in exon 18 of angiotensin-converting enzyme 2 gene and is capable of inducing exon skipping in angiotensin-converting enzyme 2 gene.

The nucleotide sequence of exon 18 of human angiotensin-converting enzyme 2 gene is shown in SEQ ID NO: 1. In the present invention, when the nucleotide sequence of exon 18 of angiotensin-converting enzyme 2 gene is the nucleotide sequence as shown in SEQ ID NO: 1, a target site in exon 18 of angiotensin-converting enzyme 2 gene may be suitably located within the region of nucleotide Nos. 1-195 of the nucleotide sequence as shown in SEQ ID NO: 1.

Further, in the present invention, the nucleotide sequence of the antisense oligonucleotide may conveniently comprise a sequence consisting of at least 15 consecutive nucleotides in any one of the sequences as shown in SEQ ID NOS: 2-17 (wherein "t" may be "u", and "u" may be "t").

The antisense oligonucleotide may have 18 bases, and the nucleotide sequence of the antisense oligonucleotide may be any one of the sequences as shown in SEQ ID NOS: 2-17 (wherein "t" may be "u", and "u" may be "t").

Nucleotides constituting the antisense oligonucleotide may be either natural DNA, natural RNA, or modified DNA or RNA. Preferably, at least one of the nucleotides is a modified nucleotide.

Examples of modified nucleotides include those in which a sugar is modified [e.g., the hydroxy group at 2'-position of D-ribofuranose is modified as in the case where D-ribofuranose is 2'-O-alkylated or 2'-O,4'-C-alkylenated], those in which a phosphodiester bond is modified (e.g., thioated), those in which a base is modified, combinations of the above-described nucleotides, and so forth. Antisense oligonucleotides in which at least one D-ribofuranose constituting the oligonucleotides is 2'-O-alkylated or 2'-O,4'-C-alkylenated have high RNA binding strength and high resistance to nuclease. Thus, they are expected to produce higher therapeutic effect than natural nucleotides (i.e. oligo DNA or oligo RNA). Further, oligonucleotides in which at least one phosphodiester bond constituting the oligonucleotides is thioated also have high resistance to nuclease and, thus, are expected to produce higher therapeutic effect than natural nucleotides (i.e. oligo DNA or oligo RNA). Oligonucleotides comprising both the modified sugar and the modified phosphate as described above have even higher resistance to nuclease and, thus, are expected to produce even higher therapeutic effect.

With respect to the antisense oligonucleotide, examples of modified sugars include, but are not limited to, D-ribofuranose as 2'-O-alkylated (e.g. 2'-O-methylated, 2'-O-aminoethylated, 2'-O-propylated, 2'-O-allylated, 2'-O-methoxyethylated, 2'-O-butylated, 2'-O-pentylated, or 2'-O-propargylated); D-ribofuranose as 2'-O,4'-C-alkylenated (e.g. 2'-O,4'-C-ethylenated, 2'-O,4'-C-methylenated, 2'-O,4'-C-propylenated, 2'-O,4'-C-tetramethylenated, or 2'-O,4'-C-pentamethylenated); 3' -deoxy-3' -amino-2' -deoxy-D-ribofuranose, 3' -deoxy-3' -amino-2' -deoxy-2'-fluoro-D-ribofuranose, etc.

With respect to the antisense oligonucleotide, examples of the modification of phosphodiester bond include, but are not limited to, phosphorothioate bond, methylphosphonate bond, methylthiophosphonate bond, phosphorodithioate bond and phosphoramidate bond.

With respect to the antisense oligonucleotide, examples of modified bases include, but are not limited to, cytosine as 5-methylated, 5-fluorinated, 5-brominated, 5-iodinated or N4-methylated; thymidine as 5-demethylated (uracil), 5-fluorinated, 5-brominated or 5-iodinated; adenine as N6-methylated or 8-brominated; guanine as N2-methylated or 8-brominated; and uridine as pseudouridinated or 1-methyl-pseudouridinated.

The antisense oligonucleotide of the present invention may be used in the form of a salt. When the antisense oligonucleotide of the present invention is used in a pharmaceutical drug, salts may suitably be pharmaceutically acceptable salts. Examples of such salts include, but are not limited to, alkaline metal salts such as sodium salts, potassium salts or lithium salts; alkaline earth metal salts such as calcium salts or magnesium salts; metal salts such as aluminum salts, iron salts, zinc salts, copper salts, nickel salts or cobalt salts; amine salts including inorganic salts such as ammonium salts and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts or tris(hydroxymethyl)aminomethane salts; inorganic acid salts including hydrohalogenic acid salts such as hydrofluorides, hydrochlorides, hydrobromides or hydroiodides, as well as nitrates, perchlorates, sulfates or phosphates; organic acid salts including lower alkane sulfonic acid salts such as methanesulfonates, trifluoromethanesulfonates or ethanesulfonates, arylsulfonic acid salts such as benzenesulfonates or p-toluenesulfonates, as well as acetates, malates, fumarates, succinates, citrates, tartrates, oxalates or maleates; and amino acid salts such as glycine salts, lysine salts, arginine salts, ornithine salts, glutamic acid salts or aspartic acid salts. These salts may be prepared by known methods.

The antisense oligonucleotide sometimes occur as a solvate (e.g., hydrate). The antisense oligonucleotide may be such a solvate.

Further, the antisense oligonucleotide may be administered in the form of a prodrug. Examples of such prodrug include, but are not limited to, amides, esters, carbamates, carbonates, ureides and phosphates. These prodrugs may be prepared by known methods.

Methods of synthesizing the antisense oligonucleotide are not particularly limited, and conventional methods may be used. Such conventional methods include, but are not limited to, synthesis methods using genetic engineering techniques and chemical synthesis methods. Genetic engineering techniques include, but are not limited to, *in vitro* transcription synthesis method, method using vectors, and method using PCR cassettes. The vector is not particularly limited. Nonviral vectors such as plasmids, virus vectors, etc. may be used. The chemical synthesis method is not particularly limited. For example, the phosphoramidite method, the H-phosphonate method, or the like may be enumerated. For the aforementioned chemical synthesis method, a commercially available automated nucleic acid synthesizer, for example, may be used. Generally, amidites are used in the chemical synthesis method. The amidite is not particularly limited. In Examples described later, antisense oligonucleotides were synthesized by the phosphoramidite method using ENA-2CE phosphoramidite and 2'OMe-2CE phosphoramidite.

As regards phosphoramidite reagents to be used, natural nucleosides and 2'-O-methylnucleosides (i.e., 2'-O-methylguanosine, 2'-O-methyladenosine, 2'-O-methylcytosine and 2'-O-methyluridine) are commercially available. As regards 2'-O-alkylguanosine, -alkyladenosine, - alkylcytosine and -alkyluridine in which the carbon number of the alkyl group is 2-6, the following methods may be employed.

2'-O-aminoethylguanosine, -aminoethyladenosine, -aminoethylcytosine and -aminoethyluridine may be synthesized as previously described in a document (Blommers et al., Biochemistry (1998), 37, 17714-17725).

2'-O-propylguanosine, -propyladenosine, -propylcytosine and -propyluridine may be synthesized as previously described in a document (Lesnik, E.A. et al., Biochemistry (1993), 32, 7832-7838).

For the synthesis of 2'-O-allylguanosine, -allyladenosine, -allylcytosine and -allyluridine, commercially available reagents may be used.

2'-O-methoxyethylguanosine, -methoxyethyladenosine, -methoxyethylcytosine and -methoxyethyluridine may be synthesized as previously described in a patent (US Patent No. 6261840) or in a document (Martin, P. Helv. Chim. Acta. (1995) 78, 486-504).

2'-O-butylguanosine, -butyladenosine, -butylcytosine and -butyluridine may be synthesized as previously described in a document (Lesnik, E.A. et al., Biochemistry (1993), 32, 7832-7838).

2'-O-pentylguanosine, -pentyladenosine, -pentylcytosine and -pentyluridine may be synthesized as previously described in a document (Lesnik, E.A. et al., Biochemistry (1993), 32, 7832-7838).

For the synthesis of 2'-O-propargylguanosine, -propargyladenosine, -propargylcytosine and -propargyluridine, commercially available reagents may be used.

2'-O,4'-C-methyleneguanosine, 2'-O,4'-C-methyleneadenosine, 5-methylcytosine and 5-methylthymidine may be prepared according to the method described in WO99/14226; and 2'-O,4'-C-alkyleneguanosine, 2'-O,4'-C-alkyleneadenosine, 5-methylcytosine and 5-methylthymidine in which the carbon number of the alkylene group is 2-5 may be prepared according to the method described in WO00/47599.

An antisense oligonucleotide with phophorothioate bonds can be synthesized by coupling phosphoramidite reagents and then reacting sulfur, tetraethylthiuram disulfide (TETD; Applied Biosystems), Beaucage reagent (Glen Research) or a reagent such as xanthan hydride (Tetrahedron Letters, 32, 3005 (1991); J. Am. Chem. Soc. 112, 1253 (1990); PCT/WO98/54198).

As controlled pore glass (CPG) to be used in a DNA synthesizer, 2'-O-methylnucleoside-bound CPG is commercially available. As regards 2'-O,4'-C-methyleneguanosine, 2'-O,4'-C-methyleneadenosine, 5-methylcytosine and 5-methylthymidine, they may be prepared according to the method described in WO99/14226; and as regards 2'-O,4'-C-alkyleneguanosine, 2'-O,4'-C-alkyleneadenosine, 5-methylcytosine and 5-methylthymidine in which the carbon number of the alkylene group is 2-5, they may be prepared according to the method described in WO00/47599. The thus prepared nucleosides may then be bound to CPG as previously described in a document (Oligonucleotide Synthesis, Edited by M. J. Gait, Oxford University Press, 1984). By using the modified CPG (as disclosed in Example 12b of Japanese Unexamined Patent Publication No. Hei7-87982), an oligonucleotide in which a 2-hydroxyethylphosphate group is bound at the 3' end can be synthesized. If 3'-amino-Modifier C3 CPG, 3'-amino-Modifier C7 CPG or Glyceryl CPG (Glen Research) or 3'-specer C3 SynBase CPG 1000 or 3'-specer C9 SynBase CPG 1000 (Link Technologies) is used, an oligonucleotide in which a hydroxyalkylphosphate group or aminoalkylphosphate group is bound at the 3' end can be synthesized.

The antisense oligonucleotide of the present invention may be used in a pharmaceutical drug. When used in a pharmaceutical drug, the antisense oligonucleotide may be in the form of a pharmaceutically acceptable salt, solvate or prodrug. Therefore, the present invention provides a pharmaceutical drug comprising an antisense oligonucleotide of 15-30 bases or a pharmaceutically acceptable salt or a solvate thereof, wherein the antisense oligonucleotide has a nucleotide sequence complementary to a target site in exon 18 of angiotensin-converting enzyme 2 gene and is capable of inducing exon skipping in angiotensin-converting enzyme 2 gene. The pharmaceutical drug may be for inhibiting the infectivity of SARS-CoV-2, but uses of the pharmaceutical drug are not limited to this particular case. The pharmaceutical drug of the present invention may have an effect of inhibiting cell entry of the virus s by decreasing receptor-type angiotensin-converting enzyme 2 and/or an effect of extracellularly capturing the virus by increasing soluble angiotensin-converting enzyme 2 which is capable of binding to the virus. The pharmaceutical drug of the present invention may be for preventing and/or treating SARS-CoV-2 infection. The present invention also provides a method for inhibiting the infectivity of SARS-CoV-2, comprising administering to a subject an effective amount of the antisense oligonucleotide or a pharmaceutically acceptable salt or a solvate thereof. The present invention also provides a method of preventing and/or treating SARS-CoV-2 infection, comprising administering to a subject an effective amount of the antisense oligonucleotide or a pharmaceutically acceptable salt or a solvate thereof. The subject may be a human or an animal. As regards the animal, mammals such as dog, cat, mink, tiger, lion, mouse, rat, rabbit, sheep, pig, cattle, horse, and the like may be enumerated.

As used herein, the term "prevention of infection" encompasses prevention of infection with viruses, prevention of the development of unfavorable symptoms caused by infection with viruses (viral infections), and prevention of the exacerbation of viral infections; and the term "prevention" encompasses reducing virus infection rate, reducing incidence rate of unfavorable symptoms caused by infection with viruses, reducing exacerbation rate of viral infections, and lowering the extent of exacerbation of viral infections.

As used herein, the term "treatment of infection" encompasses recovery from infection with viruses, amelioration of unfavorable symptoms caused by infection with viruses, and prevention or delaying of the exacerbation of viral infections.

The antisense oligonucleotide of the present invention or a pharmaceutically acceptable salt, solvate or prodrug thereof (hereinafter, referred to as "active ingredient") may be administered, either alone or together with pharmacologically acceptable carriers, diluents or excipients in appropriate forms of preparations, to mammals (e.g., human, rabbit, dog, cat, rat, mouse, etc.) orally or parenterally. Dose levels may vary depending on the subject to be treated, symptoms, administration route, and so on. For example, typically about 0.1 to 50 mg/kg body weight, preferably about 0.5 mg /kg body weight, may be administered as a dosage unit in terms of active ingredient at a frequency of about one to three times a day, preferably at a frequency of about once a day, by intranasal administration or intravenous injection (preferably administered consecutively or on alternate days). In cases of other parenteral administration and oral administration, similar dose levels may be used. If symptoms are very severe, the dosage may be increased accordingly.

Preparations for oral administration include solid or liquid preparations such as tablets (including sugar-coated tablets and film-coated tablets), pills, granules, powders, capsules (including soft capsules), syrups, emulsions and suspensions. These preparations may be prepared according to conventional methods and may contain those carriers, diluents or excipients which are conventionally used in the pharmaceutical formulation procedure. For example, lactose, starch, sucrose, magnesium stearate and the like may be used as carriers or excipients for tablets.

Preparations for parenteral administration include, for example, collunaria, injections and suppositories. Collunaria may be in such dosage forms as nasal powder or nasal drops. Injections include intravenous injections, subcutaneous injections, intradermal injections, muscle injections, instilment injections, etc. Nasal powder may be prepared by pulverizing the active ingredient into appropriately fine granules and, if necessary, mixing the granules with additives so that a homogeneous mixture is obtained. Nasal drops may be obtained by adding solvents, additives and so forth to the active ingredient, dissolving or suspending the resultant mixture, and, if necessary, filtering the resultant solution or suspension. Isotonizing agents, pH adjusters, etc. may also be used. Examples of additives include preservatives such as benzalkonium chloride, binders such as hydroxypropyl cellulose, excipients such as lactose, and the like. As the solvent, physiological saline is typically used. Auxiliary solubilizers such as alcohol (e.g., ethanol, isopropyl alcohol, etc.), glycol (e.g., propylene glycol, polyethylene glycol, polypropylene glycol, glycol ether, glycerol, etc.), polyoxyethylene alcohol, or the like may be added. Injections may be prepared by conventional methods, i.e., by dissolving, suspending or emulsifying the active ingredient in an aseptic, aqueous or oily liquid that is conventionally used in injections. Examples of aqueous liquids for injection include physiological saline and isotonic solutions containing glucose and other auxiliary agents. They may be used in combination with a suitable auxiliary solubilizer such as alcohol (e.g. ethanol), polyalcohol (e.g. propylene glycol, polyethylene glycol), nonionic surfactant [e.g. Polysorbate 80, HCO-50 (polyoxyethylene (50 mol) adduct of hydrogenated castor oil)], etc. Examples of oily liquids for injection include sesame oil and soybean oil. They may be used in combination with an auxiliary solubilizer such as benzyl benzoate, benzyl alcohol, etc. Usually, the prepared injections are filled in appropriate ampoules. Suppositories for administration into the rectum may be prepared by mixing the active ingredient with a conventional suppository base.

It is convenient to formulate the above-described pharmaceutical preparations for oral or parenteral administration into unit dosage forms that would give an appropriate dose of the active ingredient. Examples of such unit dosage forms include tablets, pills, capsules, preparations for nasal administration as filled in collunarium containers, injections (ampoules), and suppositories. It is preferred that 0.1 to 1000 mg of the active ingredient is typically contained in each unit dosage form.

The antisense oligonucleotide of the present invention makes it possible to inhibit the expression of angiotensin-converting enzyme 2 protein and/or to enhance the expression of soluble angiotensin-converting enzyme 2. Therefore, the present invention provides an agent for inhibiting the expression of angiotensin-converting enzyme 2 protein and/or enhancing the expression of soluble angiotensin-converting enzyme 2, wherein the agent comprises an antisense oligonucleotide of 15-30 bases or a salt or a solvate thereof, the antisense oligonucleotide having a nucleotide sequence complementary to a target site in exon 18 of angiotensin-converting enzyme 2 gene and being capable of inducing exon skipping in angiotensin-converting enzyme 2 gene. The agent of the present invention may be used as a pharmaceutical drug or a reagent for experiments. The present invention also provides a method of inhibiting the expression of angiotensin-converting enzyme 2 protein and/or enhancing the expression of soluble angiotensin-converting enzyme 2, comprising administering to a subject an effective amount of the above-described antisense oligonucleotide or a pharmaceutically acceptable salt or a solvate thereof. The subject may be a human or an animal. As regards the animal, mammals such as dog, cat, mink, tiger, lion, mouse, rat, rabbit, sheep, pig, cattle, horse, or the like may be enumerated. The antisense oligonucleotide may be in the form of a salt, a solvate or a prodrug. As one example of salt, solvate or prodrug, a pharmaceutically acceptable salt, solvate or prodrug may be given. For details, reference should be had to the foregoing description.

In the case of use as a reagent for experiments, the expression of angiotensin-converting enzyme 2 protein can be inhibited or the expression of soluble angiotensin-converting enzyme 2 can be enhanced by treating angiotensin-converting enzyme 2-expressing cells, tissues or organs with the antisense oligonucleotide of the present invention or a salt or a solvate thereof. The antisense oligonucleotide of the present invention or a salt or a solvate thereof may be used in an amount effective for inhibiting the expression of angiotensin-converting enzyme 2 protein or enhancing the expression of soluble angiotensin-converting enzyme 2. Examples of cells that express angiotensin-converting enzyme 2 include, but are not limited to, nasal epithelial goblet cells, type II alveolar epithelial cells and absorptive intestinal epithelial cells, liver cancer cells, vascular endothelial cells, and renal tubular epithelial cells. In addition to naturally occurring cells, - recombinant cells transfected with angiotensin-converting enzyme 2 gene may also be employed. Examples of tissues and organs that expressangiotensin-converting enzyme 2 include, but are not limited to, heart, kidney, testis, lung, testis, small intestine, kidney, and prostate. The expression of angiotensin-converting enzyme 2 may be examined by, for example, analyzing the angiotensin-converting enzyme 2 mRNA in samples by RT-PCR, or by detecting the angiotensin-converting enzyme 2 protein in samples by Western blotting or by mass spectrometry.

As a result of the exon skipping in angiotensin-converting enzyme 2 gene that has been induced by the antisense oligonucleotide of the present invention or a salt or a solvate thereof, soluble angiotensin-converting enzyme 2 lacking its transmembrane domain is produced. The present invention also provides this soluble angiotensin-converting enzyme 2.

Further, the present invention also provides a polynucleotide comprising a nucleotide sequence that encodes the soluble angiotensin-converting enzyme 2 and/or a sequence that is complementary to such nucleotide sequence.

The soluble angiotensin-converting enzyme 2 of the present invention and a polynucleotide comprising a nucleotide sequence encoding the enzyme can be produced by inducing exon skipping in angiotensin-converting enzyme 2 gene using the antisense oligonucleotide of the present invention in angiotensin-converting enzyme 2-expressing cells. Further, the soluble angiotensin-converting enzyme 2 of the present invention may be prepared as a recombinant protein by the following procedures. Briefly, RNA is extracted from cells in which the skipping of the exon of angiotensin-converting enzyme 2 gene has been induced; cDNA is synthesized using a reverse transcriptase and random primers and then amplified by PCR; sequence analysis is then conducted to determine sequences of interest; frequency of codon usage in the open reading frame of the resultant sequence is subsequently optimized; restriction enzyme recognition sequences are added to the optimized sequence on the 5' side and the 3' side, respectively; the resultant sequence is incorporated into an appropriate vector, which is then introduced into an appropriate host cell to produce a recombinant protein.

As the vector, *Escherichia coli*-derived plasmids (e.g., pBR322, pBR325, pUC12, pUC13, pUC19, pET-44, or pBlueScriptII); *Bacillus subtilis-derived* plasmids (e.g., YEp13, pYES2, YRp7, YIp5, pYAC2, pUB 110, pTP5, or pC194); yeast-derived plasmids (e.g., pSH19 or pSH15); bacteriophages such as λ phage; animal viruses such as retrovirus, adenovirus, lentivirus, adeno-associated virus, or vaccinia virus; or insect pathogen viruses such as baculovirus may be used.

The expression vector may further comprise promoters, enhancers, terminators, splicing signals, poly-A addition signals, selection markers, SV40 replication origins, and so forth.

Examples of the host cell include, but are not limited to, bacterial cells (such as *Escherichia* bacteria, *Bacillus* bacteria or *Bacillus subtilis*), fungal cells (such as yeast or *Aspergillus*), insect cells (such as S2 cells or Sf cells), animal cells (such as CHO cells, COS cells, HeLa cells, C127 cells, 3T3 cells, BHK cells or HEK 293 cells) and plant cells.

Introduction of a recombinant vector into a host cell may be performed by the methods disclosed in Molecular Cloning 2nd Edition, J. Sambrook et al., Cold Spring Harbor Lab. Press, 1989 (e.g., the calcium phosphate method, the DEAE-dextran method, transfection, microinjection, lipofection, electroporation, transduction, scrape loading, the shotgun method, etc.) or by infection.

The resultant transformant may be cultured in a medium, followed by collection of the soluble angiotensin-converting enzyme 2 from the culture. When the soluble angiotensin-converting enzyme 2 is secreted into the medium, the medium may be recovered, followed by isolation and purification of the soluble angiotensin-converting enzyme 2 from the medium. When the soluble angiotensin-converting enzyme 2 is produced within the transformed cells, the cells may be lysed, followed by isolation and purification of the soluble angiotensin-converting enzyme 2 from the cell lysate.

Isolation and purification of the soluble angiotensin-converting enzyme 2 may be performed by known methods. Known isolation/purification methods which may be used in the present invention include, but are not limited to, methods using difference in solubility (such as salting-out or solvent precipitation); methods using difference in molecular weight (such as dialysis, ultrafiltration, gel filtration or SDS-polyacrylamide gel electrophoresis); methods using difference in electric charge (such as ion exchange chromatography); methods using specific affinity (such as affinity chromatography); methods using difference in hydrophobicity (such as reversed phase high performance liquid chromatography); and methods using difference in isoelectric point (such as isoelectric focusing).

### EXAMPLES

Hereinbelow, the present invention will be described in detail with reference to the following Examples.

### [Examples 1-8] Synthesis of Antisense Oligonucleotides (ASOs)

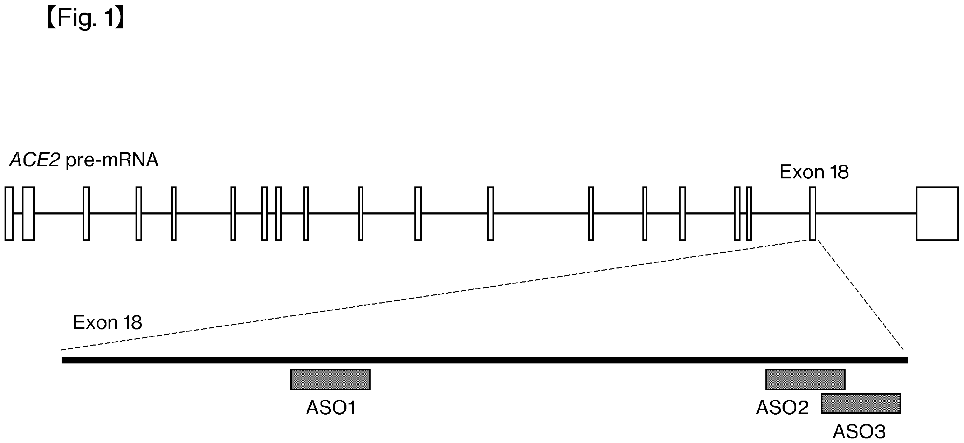

The antisense oligonucleotides (ASOs) as shown in Table 1 were synthesized. The locations of ASO sequences complementary to ACE2 pre-mRNA are shown in Figs. 1 and 3. Modified nucleic acid ENA^{®} (2'-O,4'-C-Ethylene-bridged Nucleic Acids) was introduced into A (adenine), G (guanine), C (cytosine) and T (thymine) in ASO sequences to provide improved affinity and stability.

### Synthesis of gCTgTaTCCCCagaaaCT (ACE2ASO1) (Example 1)

Synthesis was performed with an automated nucleic acid synthesizer (DNA/RNA synthesizer model NTS H-6: Nihon Techno Service Co., Ltd.) at 1 µmol scale. Concentrations of solvents, reagents and phosphoramidites in each synthesis cycle were the same as those concentrations used in natural oligonucleotide synthesis. Reagents and 2'-O-methylnucleoside phosphoramidites (for adenosine: Product No. 10-3100-10; for guanosine: Product No. 10-3121-10) were available from Glen Research. Solvents used were products from Wako Pure Chemical Industries. Non-natural phosphoramidites used were the following compounds disclosed in Japanese Unexamined Patent Publication No. 2000-297097 at Example 22 (5'-O-dimethoxytrityl-2'-O,4'-C-ethylene-4-N-benzoyl-5-methylcytidine-3'-O-(2-cyanoethyl N,N-diisopropyl)phosphoramidite) and Example 9 (5'-O-dimethoxytrityl-2'-O,4'-C-ethylene-5-methyluridine-3' -O-(2-cyanoethyl N,N-diisopropyl)phosphoramidite). As a solid carrier, universal controlled pore glass (CPG) (Product No. 25-5040; Glen Research) was used. Thus, the titled compound was synthesized. It should be noted here that 15 minutes was set as the time required for condensation of amidites.

Protected oligonucleotide analogs with the sequence of interest were thermally treated with thick aqueous ammonia (55°C, 8 hrs) to thereby cut out oligomers from the support and, at the same time, remove the protective group cyanoethyl on phosphorus atoms and the protective group on nucleobases. The resultant ammonia solution was transferred into Glen-Pak DNA Purification Cartridge (Product No. 60-5100; Glen Research), in which DMT groups were removed according to the protocol recommended by Glen Research. The recovered solution was evaporated under reduced pressure; the residue was purified by reversed phase HPLC [(LC-2a from Shimadzu Corporation; column (Triart C18 from YMC (10x150 mm)); Solution A: 0.1 M triethylamine acetate aqueous solution (TEAA), pH 7.0; Solution B: acetonitrile, B%: from 10% to 25% (30 min, linear gradient); 50°C; 4.7 mL/min; 280 nm]. After distilling off the solvent, the residue was dissolved in 10 mM NaOH solution, and pure water substitution was performed by ultrafiltration using a Microsep centrifugal filtration device (Product No. MAP003C; Nippon Pall). After lyophilization, a compound of interest was obtained.

The subject compound was identified by negative-ion MALDI-TOFMS (theoretical: 6461, found: 6461).

The nucleotide sequence of the subject compound is a sequence complementary to nucleotide Nos. 36796-36813 of Homo sapiens angiotensin I converting enzyme 2 (ACE2), RefSeqGene on chromosome X (Gene Bank accession No. NG_012575.1).

### Synthesis of gaTCCCagTgaagaTCag (ACE2ASO2) (Example 2)

The compound of Example 2 with the sequence of interest was synthesized in the same manner as described for the compound of Example 1.

The subject compound was identified by negative-ion MALDI-TOFMS (theoretical: 6486, found: 6487).

The nucleotide sequence of the subject compound is a sequence complementary to nucleotide Nos. 36906-36923 of Homo sapiens angiotensin I converting enzyme 2 (ACE2), RefSeqGene on chromosome X (Gene Bank accession No. NG_012575.1).

### Synthesis of TCTTCCgaTCTCTgaTCC (ACE2ASO3) (Example 3)

The compound of Example 3 with the sequence of interest was synthesized in the same manner as described for the compound of Example 1.

The subject compound was identified by negative-ion MALDI-TOFMS (theoretical: 6456, found: 6456).

The nucleotide sequence of the subject compound is a sequence complementary to nucleotide Nos. 36919-36936 of Homo sapiens angiotensin I converting enzyme 2 (ACE2), RefSeqGene on chromosome X (Gene Bank accession No. NG_012575.1).

### Synthesis of TgaTaCggCTCCgggaCa (ACE2ASO4) (Example 4)

The compound of Example 4 with the sequence of interest was synthesized in the same manner as described for the compound of Example 1.

The subject compound was identified by negative-ion MALDI-TOFMS (theoretical: 6504, found: 6504).

The nucleotide sequence of the subject compound is a sequence complementary to nucleotide Nos. 36745-36762 of Homo sapiens angiotensin I converting enzyme 2 (ACE2), RefSeqGene on chromosome X (Gene Bank accession No. NG_012575.1).

### Synthesis of ggCTgTTgTCaTTCagaC (ACE2ASO5) (Example 5)

The compound of Example 5 with the sequence of interest was synthesized in the same manner as described for the compound of Example 1.

The subject compound was identified by negative-ion MALDI-TOFMS (theoretical: 6495, found: 6495).

The nucleotide sequence of the subject compound is a sequence complementary to nucleotide Nos. 36775-36792 of Homo sapiens angiotensin I converting enzyme 2 (ACE2), RefSeqGene on chromosome X (Gene Bank accession No. NG_012575.1).

### Synthesis of TaggaggTCCaagTgTTg (ACE2ASO6) (Example 6)

The compound of Example 6 with the sequence of interest was synthesized in the same manner as described for the compound of Example 1.

The subject compound was identified by negative-ion MALDI-TOFMS (theoretical: 6520, found: 6521).

The nucleotide sequence of the subject compound is a sequence complementary to nucleotide Nos. 36814-36831 of Homo sapiens angiotensin I converting enzyme 2 (ACE2), RefSeqGene on chromosome X (Gene Bank accession No. NG_012575.1).

### Synthesis of CCaTaTggaaaCaggggg (ACE2ASO7) (Example 7)

The compound of Example 7 with the sequence of interest was synthesized in the same manner as described for the compound of Example 1.

The subject compound was identified by negative-ion MALDI-TOFMS (theoretical: 6513, found: 6513).

The nucleotide sequence of the subject compound is a sequence complementary to nucleotide Nos. 36837-36854 of Homo sapiens angiotensin I converting enzyme 2 (ACE2), RefSeqGene on chromosome X (Gene Bank accession No. NG_012575.1).

### Synthesis of CaCaaCTCCaaaaaCaaT (ACE2ASO8) (Example 8)

The compound of Example 8 with the sequence of interest was synthesized in the same manner as described for the compound of Example 1.

The subject compound was identified by negative-ion MALDI-TOFMS (theoretical: 6407, found: 6407).

The nucleotide sequence of the subject compound is a sequence complementary to nucleotide Nos. 36858-36875 of Homo sapiens angiotensin I converting enzyme 2 (ACE2), RefSeqGene on chromosome X (Gene Bank accession No. NG_012575.1).

### Synthesis of aaTgCCaaCCaCTaTCaC (ACE2ASO9) (Example 9)

The compound of Example 9 with the sequence of interest was synthesized in the same manner as described for the compound of Example 1.

The subject compound was identified by negative-ion MALDI-TOFMS (theoretical: 6428, found: 6429).

The nucleotide sequence of the subject compound is a sequence complementary to nucleotide Nos. 36882-36899 of Homo sapiens angiotensin I converting enzyme 2 (ACE2), RefSeqGene on chromosome X (Gene Bank accession No. NG_012575.1).

### Synthesis of CgggaCaTCcTaTTTgCa (ACE2ASOIn16Ex17) (Example 10)

The compound of Example 10 with the sequence of interest was synthesized in the same manner as described for the compound of Example 1.

The subject compound was identified by negative-ion MALDI-TOFMS (theoretical: 6452, found: 6452).

The nucleotide sequence of the subject compound is a sequence complementary to nucleotide Nos. 36734-36751 of Homo sapiens angiotensin I converting enzyme 2 (ACE2), RefSeqGene on chromosome X (Gene Bank accession No. NG_012575.1).

### Synthesis of gCCacTTacTTcTTCCga (ACE2ASOEx17In17) (Example 11)

The compound of Example 11 with the sequence of interest was synthesized in the same manner as described for the compound of Example 1.

The subject compound was identified by negative-ion MALDI-TOFMS (theoretical: 6375, found: 6377).

The nucleotide sequence of the subject compound is a sequence complementary to nucleotide Nos.36929-36946 of Homo sapiens angiotensin I converting enzyme 2 (ACE2), RefSeqGene on chromosome X (Gene Bank accession No. NG_012575.1).

### Synthesis of CggCTCCgggaCaTccTa (ACE2ASO4+5) (Example 12)

The compound of Example 12 with the sequence of interest was synthesized in the same manner as described for the compound of Example 1.

The subject compound was identified by negative-ion MALDI-TOFMS (theoretical: 6440, found: 6440).

The nucleotide sequence of the subject compound is a sequence complementary to nucleotide Nos. 36740-36757 of Homo sapiens angiotensin I converting enzyme 2 (ACE2), RefSeqGene on chromosome X (Gene Bank accession No. NG_012575.1).

### Synthesis of CggaAAgcATCaTTgaTA (ACE2ASO4-13) (Example 13)

The compound of Example 13 with the sequence of interest was synthesized in the same manner as described for the compound of Example 1.

The subject compound was identified by negative-ion MALDI-TOFMS (theoretical: 6493, found: 6492).

The nucleotide sequence of the subject compound is a sequence complementary to nucleotide Nos. 36758-36775 of Homo sapiens angiotensin I converting enzyme 2 (ACE2), RefSeqGene on chromosome X (Gene Bank accession No. NG_012575.1).

### Synthesis of CTCTaggcTgTTgTcaTT (ACE2ASO5-5) (Example 14)

The compound of Example 14 with the sequence of interest was synthesized in the same manner as described for the compound of Example 1.

The subject compound was identified by negative-ion MALDI-TOFMS (theoretical: 6433, found: 6434).

The nucleotide sequence of the subject compound is a sequence complementary to nucleotide Nos. 36780-36797 of Homo sapiens angiotensin I converting enzyme 2 (ACE2), RefSeqGene on chromosome X (Gene Bank accession No. NG_012575.1).

### Synthesis of TCcaagTgTTggCTgTAT (ACE2ASO6+7) (Example 15)

The compound of Example 15 with the sequence of interest was synthesized in the same manner as described for the compound of Example 1.

The subject compound was identified by negative-ion MALDI-TOFMS (theoretical: 6482, found: 6482).

The nucleotide sequence of the subject compound is a sequence complementary to nucleotide Nos. 36807-36824 of Homo sapiens angiotensin I converting enzyme 2 (ACE2), RefSeqGene on chromosome X (Gene Bank accession No. NG_012575.1).

### Synthesis of AgggggcTGgTTAggagG (ACE2ASO6-11) (Example 16)

The compound of Example 16 with the sequence of interest was synthesized in the same manner as described for the compound of Example 1.

The subject compound was identified by negative-ion MALDI-TOFMS (theoretical: 6482, found: 6482).

The nucleotide sequence of the subject compound is a sequence complementary to nucleotide Nos. 36825-36842 of Homo sapiens angiotensin I converting enzyme 2 (ACE2), RefSeqGene on chromosome X (Gene Bank accession No. NG_012575.1).

### Synthesis of TTgTCaTTCagaCggaaa (ACE2ASO5+5) (Example 17)

The compound of Example 17 with the sequence of interest was synthesized in the same manner as described for the compound of Example 1.

The subject compound was identified by negative-ion MALDI-TOFMS (theoretical: 6474, found: 6474).

The nucleotide sequence of the subject compound is a sequence complementary to nucleotide Nos. 36770-36787 of Homo sapiens angiotensin I converting enzyme 2 (ACE2), RefSeqGene on chromosome X (Gene Bank accession No. NG_012575.1).

### Synthesis of TgTCaTTCagaCggaaag (ACE2ASO5+6) (Example 18)

The compound of Example 18 with the sequence of interest was synthesized in the same manner as described for the compound of Example 1.

The subject compound was identified by negative-ion MALDI-TOFMS (theoretical: 6487, found: 6489).

The nucleotide sequence of the subject compound is a sequence complementary to nucleotide Nos. 36769-36786 of Homo sapiens angiotensin I converting enzyme 2 (ACE2), RefSeqGene on chromosome X (Gene Bank accession No. NG_012575.1).

### Synthesis of gTCaTTCagaCggaaagC (ACE2ASO5+7) (Example 19)

The compound of Example 19 with the sequence of interest was synthesized in the same manner as described for the compound of Example 1.

The subject compound was identified by negative-ion MALDI-TOFMS (theoretical: 6486, found: 6488).

The nucleotide sequence of the subject compound is a sequence complementary to nucleotide Nos. 36768-36785 of Homo sapiens angiotensin I converting enzyme 2 (ACE2), RefSeqGene on chromosome X (Gene Bank accession No. NG_012575.1).

### Synthesis of TCaTTCagaCggaaagCa (ACE2ASO5+8) (Example 20)

The compound of Example 20 with the sequence of interest was synthesized in the same manner as described for the compound of Example 1.

The subject compound was identified by negative-ion MALDI-TOFMS (theoretical: 6470, found: 6474).

The nucleotide sequence of the subject compound is a sequence complementary to nucleotide Nos. 36767-36784 of Homo sapiens angiotensin I converting enzyme 2 (ACE2), RefSeqGene on chromosome X (Gene Bank accession No. NG_012575.1).

### Synthesis of aTTCagaCggaaagCaTC (ACE2ASO5+10) (Example 21)

The compound of Example 21with the sequence of interest was synthesized in the same manner as described for the compound of Example 1.

The subject compound was identified by negative-ion MALDI-TOFMS (theoretical: 6470, found: 6472).

The nucleotide sequence of the subject compound is a sequence complementary to nucleotide Nos. 36765-36782 of Homo sapiens angiotensin I converting enzyme 2 (ACE2), RefSeqGene on chromosome X (Gene Bank accession No. NG_012575.1).

### Synthesis of TCagaCggaaagCaTCaT (ACE2ASO5+12) (Example 22)

The compound of Example 22 with the sequence of interest was synthesized in the same manner as described for the compound of Example 1.

The subject compound was identified by negative-ion MALDI-TOFMS (theoretical: 6470, found: 6472).

The nucleotide sequence of the subject compound is a sequence complementary to nucleotide Nos. 36763-36780 of Homo sapiens angiotensin I converting enzyme 2 (ACE2), RefSeqGene on chromosome X (Gene Bank accession No. NG_012575.1).

### Synthesis of GgcTgtTgtCaTtCagaC (ACE2ASOSc) (Example 23)

The compound of Example 23 with the sequence of interest was synthesized in the same manner as described for the compound of Example 1.

The subject compound was identified by negative-ion MALDI-TOFMS (theoretical: 6403, found: 6399).

The nucleotide sequence of the subject compound is a sequence complementary to nucleotide Nos. 36775-36792 of Homo sapiens angiotensin I converting enzyme 2 (ACE2), RefSeqGene on chromosome X (Gene Bank accession No. NG_012575.1).

**(Table 1)**

| Sequences of the ASOs synthesized in Examples are shown. Capital letters represent ENA nucleic acid and small letters 2'OMe. | | | |
|---|---|---|---|
| Example | Designation | Sequence | SEQ ID NO: |
| 1 | ACE2ASO1 | gCTgTaTCCCCagaaaCT | 2 |
| 2 | ACE2ASO2 | gaTCCCagTgaagaTCag | 3 |
| 3 | ACE2ASO3 | TCTTCCgaTCTCTgaTCC | 4 |
| 4 | ACE2ASO4 | TgaTaCggCTCCgggaCa | 5 |
| 5 | ACE2ASO5 | ggCTgTTgTCaTTCagaC | 6 |
| 6 | ACE2ASO6 | TaggaggTCCaagTgTTg | 7 |
| 7 | ACE2ASO7 | CCaTaTggaaaCaggggg | 8 |
| 8 | ACE2ASO8 | CaCaaCTCCaaaaaCaaT | 9 |
| 9 | ACE2ASO9 | aaTgCCaaCCaCTaTCaC | 10 |
| 10 | ACE2ASOIn16Ex17 | CgggaCaTCcTaTTTgCa | 11 |
| 11 | ACE2ASOEx17In17 | gCCacTTacTTcTTCCga | 12 |
| 12 | ACE2ASO4+5 | CggCTCCgggaCaTccTa | 13 |
| 13 | ACE2ASO4-13 | CggaAAgcATCaTTgaTA | 14 |
| 14 | ACE2ASO5-5 | CTCTaggcTgTTgTcaTT | 15 |
| 15 | ACE2ASO6+7 | TCcaagTgTTggCTgTAT | 16 |
| 16 | ACE2ASO6-11 | AgggggcTGgTTAggagG | 17 |
| 17 | ACE2ASO5+5 | TTgTCaTTCagaCggaaa | 22 |
| 18 | ACE2ASO5+6 | TgTCaTTCagaCggaaag | 23 |
| 19 | ACE2ASO5+7 | gTCaTTCagaCggaaagC | 24 |
| 20 | ACE2ASO5+8 | TCaTTCagaCggaaagCa | 25 |
| 21 | ACE2ASO5+10 | aTTCagaCggaaagCaTC | 26 |
| 22 | ACE2ASO5+12 | TCagaCggaaagCaTCaT | 27 |
| 23 | ACE2ASO5c | GgcTgtTgtCaTtCagaC | 28 |

### [Test Example]

### Experimental Methods

### Evaluation of ACE2 mRNA

Changes in the splicing pattern of ACE2 caused by ASO introduction were evaluated by RT-PCR using human liver carcinoma cells (HepG2, ATCC).

### Cell Culture

Human liver carcinoma cells (HepG2) were cultured in 10% FBS (10270-106: Gibco)-containing E-MEM medium (051-07615: Fujifilm Wako Pure Chemical Corporation).

### ASO Transfection

1) Two microliters of each ASO (adjusted to a concentration of 50 pmol/µl with Nuclease-Free Water (AM9932, Thermo Fisher Scientific)) was mixed with 100 µl of Opti-MEM medium (31985070, Thermo Fisher Scientific). For control (without ASO treatment), 2 µl of Nuclease-Free Water was mixed with the medium.
2) In a separate tube, 4 µl of Lipofectamine^{™} 3000 Transfection Reagent (L3000015, Thermo Fisher Scientific) was mixed with 100 µl of Opti-MEM medium.
3) Liquid from 1) and liquid from 2) were mixed and left at room temperature for 15 min.
4) Human liver carcinoma cells (HepG2) cultured in 12-well plates were washed with PBS once. Then, 800 µl of Opti-MEM medium was added to each well.
5) Liquid from 3) was added to the cells of 4) above (final concentration of ASO: 100 nM), and the cells were cultured at 37°C under 5% CO₂ for 3 hrs. Then, the medium was changed to E-MEM medium containing 10% FBS, and culture was further continued.

### Preparation of RNA

1) Cells transfected with each ASO were cultured for 24 hrs and washed with PBS once. Then, 300 µl of RNA extraction reagent in High Pure RNA Isolation Kit (#11828665001, Roche Life Science) was added to the cells.
2) After leaving the cells at room temperature for 10 min, the RNA extraction reagent in each well was collected into a tube.
3) RNA was extracted according to the protocol of High Pure RNA Isolation Kit. Finally, 50 µl of RNA lysate was obtained.

### Reverse Transcription Reaction

1) To 500 ng of RNA, Random primers (#48190011, Thermo Fisher Scientific) and dNTP Mixture (2.5 mM each) (#4030, Takara) were added. The resultant solution was incubated at 65°C for 5 min and at 25°C for 10 min.
2) To the reaction mixture of 1), M-MLV Reverse Transcriptase (#28025013, Thermo Fisher Scientific), RNaseOUT^{™} Recombinant Ribonuclease Inhibitor (#10777-019, Thermo Fisher Scientific), DTT (attached to M-MLV) and 5x First Strand Buffer (attached to M-MLV) were added. The resultant reaction mixture was incubated at 37°C for 55 min and at 70°C for 10 min to thereby obtain cDNA.

### PCR Reaction and Confirmation of Reaction Products

1) To 2 µl of the resultant cDNA, 1 µl of primer ACE2F2 (5'-ctgttccgatcatctgttgc-3': SEQ ID NO: 18), 1 µl of primer ACE2R2 (5'-gagaccaaatacacactttccc-3': SEQ ID NO: 19), 0.1 µl of TaKaRa Ex Taq^{®} DNA polymerase (#RR001A, Takara), 1.6 µl of dNTP Mixture (2.5 mM each), 2 µl of 10× Ex Taq Buffer and 12.3 µl of Nuclease-Free Water were added.
2) The resultant mixture was heated at 94°C for 3 min.
3) A treatment consisting of 94°C 0.5 min, 60°C 0.5 min and 72°C 1.5 min was performed through 30 cycles.
4) Finally, the reaction mixture was heated at 72°C for 3 min.
5) The PCR products were electrophoresed and quantified with Agilent2100 bioanalyzer electrophoresis system (Agilent Technology).
6) Above operations 1) to 5) were performed on GAPDH using primer GAPDH H_F (5'-cccttcattgacctcaac-3': SEQ ID NO: 20) and primer GAPDH H_R (5'-ttcacacccatgacgaac-3': SEQ ID NO: 21, provided that operation 3) was performed through 18 cycles.

### Experimental Results

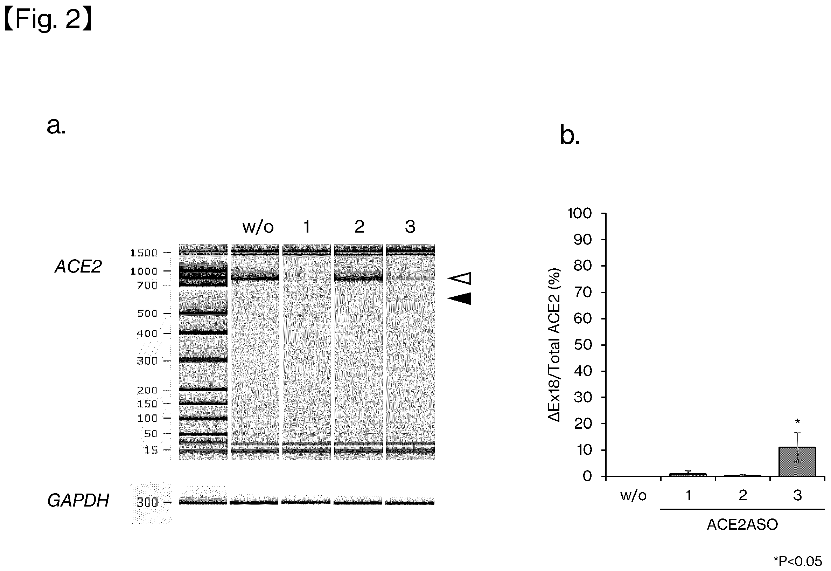

For the purpose of skipping exon 18 of ACE2 gene, the present inventors prepared 23 ASOs of 18 bases having a sequence complementary to exon 18 of ACE2 pre-mRNA (Figs. 1, 3, 5, 7, 9, 11, and 13). Each ASO was designed based on the prediction of binding of a splicing factor in ACE2 pre-mRNA. Human liver carcinoma cells (HepG2) were treated with each ASO for 24 hrs and the ACE2 mRNA levels were subsequently examined by RT-PCR. As a result, a significant increase in the ratio of exon 18-skipped ACE2 mRNA to total ACE2 mRNA was observed when cells were treated with ACE2ASO3, ACE2ASO4+5, ACE2ASO4, ACE2ASO4-13, ACE2ASO5, ACE2ASO5-5, ACE2ASO5+5, ACE2ASO5+7, ACE2ASO5+8, ACE2ASO5+10, ACE2ASO5+12 or ACE2ASO5c (Figs. 2, 4, 8, 10, 12, and 14).

### Discussion

Since ACE2 is necessary for virus infection as a receptor for virus, inhibition of the receptor function of ACE2 is attracting attention as an antiviral preventive therapy. Indeed, it has been reported that virus infection is reduced when the function of ACE2 is inhibited by acting ACE2 antibodies or peptides in cells. Further, a method has been validated in which virus infection is inhibited by decreasing ACE2 expression through the use of siRNA. ACE2 is composed of 18 exons; and it was predicted that skipping of exon 18 could produce an ACE2 protein in which the transmembrane domain is eliminated. Thus, the present inventors aimed at skipping exon 18 of ACE2 with the use of ASO to thereby reduce receptor-type ACE2 and increase free ACE2 as a result of elimination of the transmembrane domain. Consequently, a dual effect can be expected: an effect of inhibiting viral entry into cells by decrease of receptor-type ACE2 and an effect of extracellular virus capture by increase of free ACE2 that is capable of binding to the virus. Since ACE2ASO3, ACE2ASO4+5, ACE2ASO4, ACE2ASO4-13, ACE2ASO5, ACE2ASO5-5, ACE2ASO5+5, ACE2ASO5+7, ACE2ASO5+8, ACE2ASO5+10, ACE2ASO5+12 and ACE2ASO5c of the present invention increased the ratio of exon 18-skipped ACE2 mRNA to total ACE2 mRNA, these ASOs are expected to exert efficacy for preventing virus infection.

All publications, patents and patent applications cited herein are incorporated herein by reference in their entirety.

### INDUSTRIAL APPLICABILITY

The present invention is applicable as a preventive therapy using ASO. It has been revealed that ASO induces exon skipping in ACE2 gene. From this result, ASO is expected to 1) inhibit viral entry into the body through the reduction of ACE2 protein and 2) exert virus neutralization effect and Ang (1-7) effect through the production of a decoy receptor for the virus (soluble ACE2). This leads to the possibility of intranasal administration for prevention and/or treatment of virus infection, and intravenous injection as a therapy for preventing exacerbation of infection. Advantages of prevention/treatment using this ASO are:
1. Administration routes are easy to access when a residual drug evaluation at the administration site is required.
2. The effect is dual: 1) decrease in ACE2 protein, 2) increase in a decoy receptor for the virus, and 3) increase in Ang (1-7).
3. Synthesis in large quantity is possible.

### [SEQUENCE LISTING FREE TEXT]

<SEQ ID NO: 1> shows the nucleotide sequence of exon 18 of angiotensin-converting enzyme 2 gene. 
gatgtcccggagccgtatcaatgatgctttccgtctgaatgacaacagcctagagtttctggggatacagccaacacttggacctcctaaccagcccc
ctgtttccatatggctgattgtttttggagttgtgatgggagtgatagtggttggcattgtcatcctgatcttcactgggatcagagatcggaagaa
<SEQ ID NOS: 2-17 and 22-28> show nucleotide sequences of ASOs synthesized in Examples. Nucleotides constituting the antisense oligonucleotide may be either natural DNA, natural RNA, chimeric DNA/RNA, or modified DNA, RNA or DNA/RNA. Preferably, at least one of the nucleotides is a modified nucleotide.
<SEQ ID NOS: 18-21> show the nucleotide sequences of the primers used in Test Example.

## Claims

1. An antisense oligonucleotide of 15-30 bases or a salt or a solvate thereof, wherein the antisense oligonucleotide has a nucleotide sequence complementary to a target site in exon 18 of angiotensin-converting enzyme 2 gene and is capable of inducing exon skipping in angiotensin-converting enzyme 2 gene.

2. The antisense oligonucleotide or a salt or a solvate thereof of claim 1, wherein the nucleotide sequence of exon 18 of angiotensin-converting enzyme 2 gene is the nucleotide sequence as shown in SEQ ID NO: 1, and the target site in exon 18 of angiotensin-converting enzyme 2 gene is located within the region of nucleotide Nos. 1-195 of the nucleotide sequence as shown in SEQ ID NO: 1.

3. The antisense oligonucleotide or a salt or a solvate thereof of claim 1 or 2, wherein the nucleotide sequence of the antisense oligonucleotide comprises a sequence consisting of at least 15 consecutive nucleotides in any one of the sequences as shown in SEQ ID NOS: 2-17 (wherein "t" may be "u", and "u" may be "t").

4. The antisense oligonucleotide or a salt or a solvate thereof of any one of claims 1 to 3, wherein the antisense oligonucleotide has 18 bases.

5. The antisense oligonucleotide or a salt or a solvate thereof of claim 4, wherein the nucleotide sequence of the antisense oligonucleotide is any one of the sequences as shown in SEQ ID NOS: 2-17 (wherein "t" may be "u", and "u" may be "t").

6. The antisense oligonucleotide or a salt or a solvate thereof of any one of claims 1 to 5, wherein at least one nucleotide is modified.

7. The antisense oligonucleotide or a salt or a solvate thereof of claim 6, wherein the sugar constituting the modified nucleotide is D-ribofuranose and the hydroxy group at 2'-position of D-ribofuranose is modified.

8. The antisense oligonucleotide or a salt or a solvate thereof of claim 7, wherein D-ribofuranose is 2'-O-alkylated and/or 2'-O,4'-C-alkylenated.

9. A pharmaceutical drug comprising the antisense oligonucleotide of any one of claims 1 to 8 or a pharmaceutically acceptable salt or solvate thereof.

10. The pharmaceutical drug of claim 9 for inhibiting the infectivity of SARS-CoV-2.

11. The pharmaceutical drug of claim 10, which has an effect of inhibiting cell entry of the virus by decreasing receptor-type angiotensin-converting enzyme 2 and/or an effect of extracellular capturing of the virus by increasing soluble angiotensin-converting enzyme 2 capable of binding to the virus.

12. The pharmaceutical drug of any one of claims 8 to 11 for preventing and/or treating SARS-CoV-2 infection.

13. An agent for inhibiting the expression of angiotensin-converting enzyme 2 protein and/or enhancing the expression of soluble angiotensin-converting enzyme 2, wherein the agent comprises the antisense oligonucleotide or a salt or a solvate thereof of any one of claims 1 to 8.

14. A soluble angiotensin-converting enzyme 2 lacking its transmembrane domain, which is produced by exon skipping in angiotensin-converting enzyme 2 gene, said exon skipping being induced by the antisense oligonucleotide or a salt or a solvate thereof of any one of claims 1 to 8.

15. A polynucleotide comprising a nucleotide sequence encoding the soluble angiotensin-converting enzyme 2 of claim 14 and/or a sequence complementary to said sequence.

16. A method of inhibiting the infectivity of SARS-CoV-2, comprising administering to a subject an effective amount of the antisense oligonucleotide or a pharmaceutically acceptable salt or a solvate thereof of any one of claims 1 to 8.

17. A method of preventing and/or treating SARS-CoV-2 infection, comprising administering to a subject an effective amount of the antisense oligonucleotide or a pharmaceutically acceptable salt or a solvate thereof of any one of claims 1 to 8.

18. A method of inhibiting the expression of angiotensin-converting enzyme 2 protein and/or enhancing the expression of soluble angiotensin-converting enzyme 2, comprising administering to a subject an effective amount of the antisense oligonucleotide or a pharmaceutically acceptable salt or a solvate thereof of any one of claims 1 to 8.

19. The antisense oligonucleotide or a pharmaceutically acceptable salt or a solvate thereof of any one of claims 1 to 8, for use in a method of inhibiting the infectivity of SARS-CoV-2.

20. The antisense oligonucleotide or a pharmaceutically acceptable salt or a solvate thereof of any one of claims 1 to 8, for use in a method of preventing and/or treating SARS-CoV-2 infection.

21. The antisense oligonucleotide or a pharmaceutically acceptable salt or a solvate thereof of any one of claims 1 to 8, for use in a method of inhibiting the expression of angiotensin-converting enzyme 2 protein and/or enhancing the expression of soluble angiotensin-converting enzyme 2.

22. Use of the antisense oligonucleotide or a pharmaceutically acceptable salt or a solvate thereof of any one of claims 1 to 8 in the manufacture of a pharmaceutical drug for inhibiting the infectivity of SARS-CoV-2.

23. Use of the antisense oligonucleotide or a pharmaceutically acceptable salt or a solvate thereof of any one of claims 1 to 8 in the manufacture of a pharmaceutical drug for preventing and/or treating SARS-CoV-2 infection.

24. Use of the antisense oligonucleotide or a pharmaceutically acceptable salt or a solvate thereof of any one of claims 1 to 8 in the manufacture of an agent for inhibiting the expression of angiotensin-converting enzyme 2 protein and/or enhancing the expression of soluble angiotensin-converting enzyme 2.
